# EUROPEAN PATENT SPECIFICATION

(11) **EP 0 824 021 B1**
(45) Date of publication and mention of the grant of the patent: **02.04.2003**
(21) Application number: 97113409.3
(22) Date of filing: 04.08.1997
(51) Int. Cl.: A61M 1/16, G05D 11/00

(54) **Dialysis unit**
Dialyseeinheit
Unité de dialyse

(30) Priority: 06.08.1996 IT TO960677
(43) Date of publication of application: 18.02.1998
(73) Proprietor: BELLCO S.p.A., 41037 Mirandola (Modena) (IT)
(72) Inventor: Cianciavicchia, Domenico, 64040 Cavuccio (IT); Morselli, Massimo, 41038 S. Felice Sul Panaro (IT)
(74) Representative: Cerbaro, Elena, Dr.

(56) References cited:
- EP-A- 0 052 008
- US-A- 4 172 033
- US-A- 4 676 905

## Description

The present invention relates to a haemodialysis unit.

A major problem in dialysis treatment is that of setting a given weight loss of the patient during treatment.

The equipment used for this purpose normally comprises a number of noninvasive peristaltic pumps for circulating the various fluids involved, and which are nearly always associated with a number of devices for determining the flow rate of the fluids.

If the peristaltic pumps were accurate enough, the ancillary devices could be dispensed with, and the weight loss of the patient be determined by simply determining the speed of the various pumps. As it is, the precision of the peristaltic pumps is altogether insufficient to set and retroactively determine, to the required degree of accuracy, the weight loss of the patient, which, as stated, is a vital parameter which must be monitored carefully throughout dialysis treatment to prevent any possibility of cardiac unbalance.

US-A-4676905 discloses a unit for dialysation of blood comprising a dialyzer in which a membrane defines two channels, a first for a dialysis fluid and a second for extracorporeal blood; a first conduit along which the dialysis fluid flows to the first channel of the dialyzer; a second conduit along which the dialysis fluid flows from the first channel of the dialyzer; and at least two piston pumps, each of which is arranged along a closed dialysate circuit and is operated for alternatively supplying the dialysis fluid to the first conduit and for discharging the dialysis fluid along the second conduit.

However, the unit disclosed in US-A-4676905 has a relatively low precision in calculating a weight loss of a patient undergoing the treatment of dialysis.

It is an object of the present invention to provide a dialysis unit designed to overcome the aforementioned drawbacks, i.e. which provides for highly accurate control of the weight loss of the patient.

According to the present invention, there is provided a dialysis unit as recited in Claim 1.

A preferred, non-limiting embodiment of the present invention will be described by way of example with reference to the accompanying drawings, in which:
Figure 1 shows an overall diagram of the unit according to the present invention;
Figure 2 shows a side view of a number of elements forming part of the Figure 1 unit;
Figure 3 shows a plan view of the Figure 2 elements;
Figure 4 shows a side view of means for angularly displacing at least one piston pump;
Figure 5 shows a plan view of the angular displacement means in Figure 4.

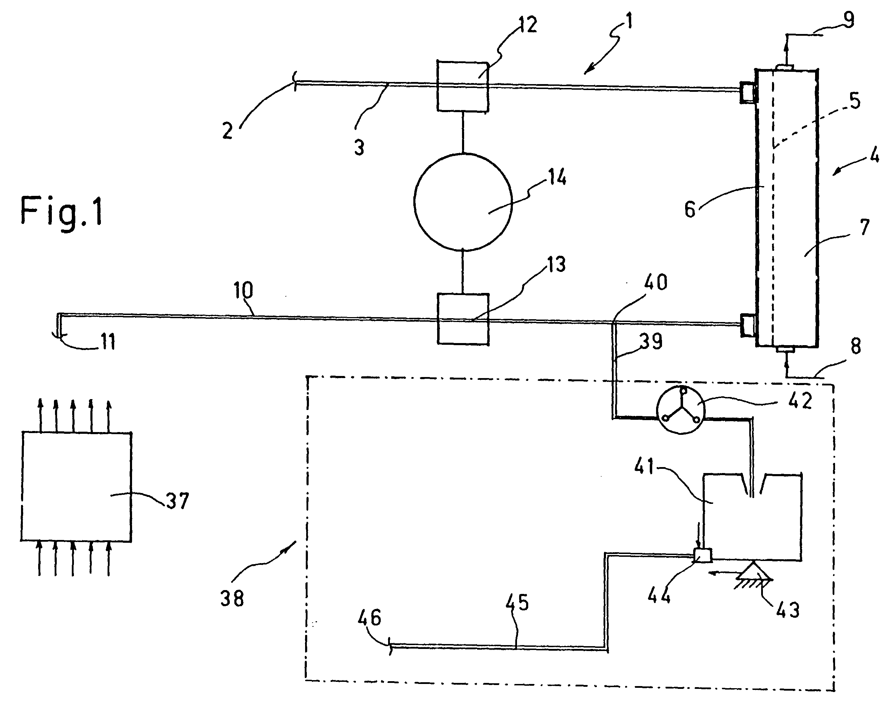

Number 1 in Figure 1 indicates as a whole a number of elements forming part of the dialysis unit according to the present invention.

In Figure 1, any details not essential to a clear understanding of the present invention have been omitted. In particular, Figure 1 does not show the first section for preparing the dialysis fluid, and which is located upstream from an inlet 2 and from a first conduit 3 for feeding the dialysis fluid into dialyzer 4.

Dialyzer 4, as is known, represents what might be called the "heart" of unit 1 by actually purifying the patient's blood, and houses a permeable membrane 5 by which it is divided longitudinally in the fluid flow direction into two parts respectively defining a first channel 6 along which the incoming dialysis fluid from first conduit 3 flows, and a second channel 7 along which the patient's blood flows from a second conduit 8, and from which the purified blood is fed out along a third conduit 9.

The undesired substances in the patient's blood diffuse through permeable membrane 5 into the dialysis fluid, by which they are fed along a fourth conduit 10 to a drain 11.

The essential elements of the present invention comprise two piston pumps 12, 13, which respectively act on the dialysis fluid flowing along first conduit 3 prior to treatment, and along fourth conduit 10 after treatment.

For the system to operate at its best, it is essential that, while the first piston pump 12 compresses, i.e. "pushes", the dialysis fluid, the second piston pump 13 draws in the dialysis fluid containing the undesired substances removed from the blood flowing through dialyzer 4.

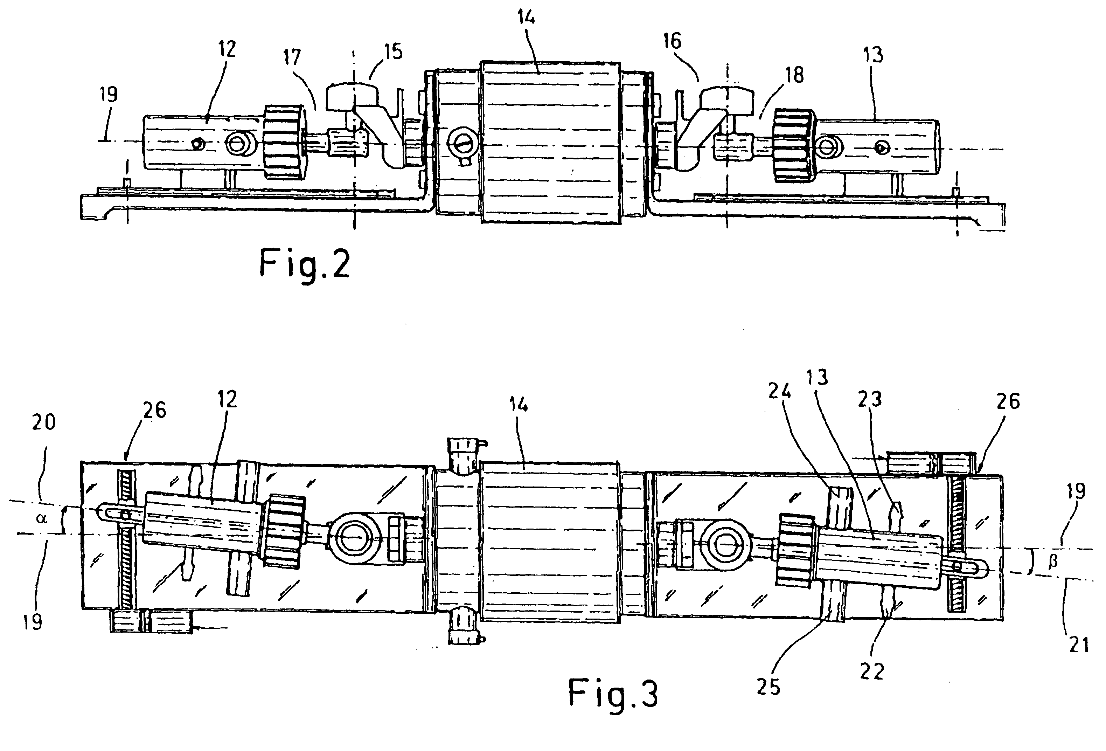

To solve the problem of setting the weight loss of the patient, the elements for performing the above functions are arranged as shown in Figures 2 and 3.

With reference to Figures 2 and 3, piston pumps 12 and 13 are located on either side of electric motor 14, which, via respective cranks 15 and 16, powers respective universal joints 17 and 18 connecting piston pumps 12 and 13 to cranks 15 and 16. As shown in Figure 3, the longitudinal axis of symmetry 19 of electric motor 14 forms an angle α with the longitudinal axis of symmetry 20 of first piston pump 12, and an angle β with the longitudinal axis of symmetry 21 of second piston pump 13.

As a result of angles α and β, universal joints 17, 18 not only transmit rotary movement to the pistons (not shown) of respective pumps 12, 13, but also move the pistons in the directions indicated by the longitudinal axes of symmetry 20, 21 of respective pumps 12, 13, which are offset with respect to the longitudinal axis of symmetry 19 of electric motor 14, so that, while first piston pump 12 compresses the fluid, second piston pump 13 draws the fluid in, thus ensuring continuous flow between pumps 12 and 13 and through dialyzer 4.

To wash piston pumps 12 and 13, the dialysis fluid entering each pump (only shown relative to piston pump 13 in Figure 3) is fed into the cylinder along a tube 22, is fed out of the cylinder along a tube 23, after being subjected to the action of the active surface of the piston (not shown), and is then fed along tubes 24 and 25 to wash the rear chamber defined by the piston surface opposite the active surface.

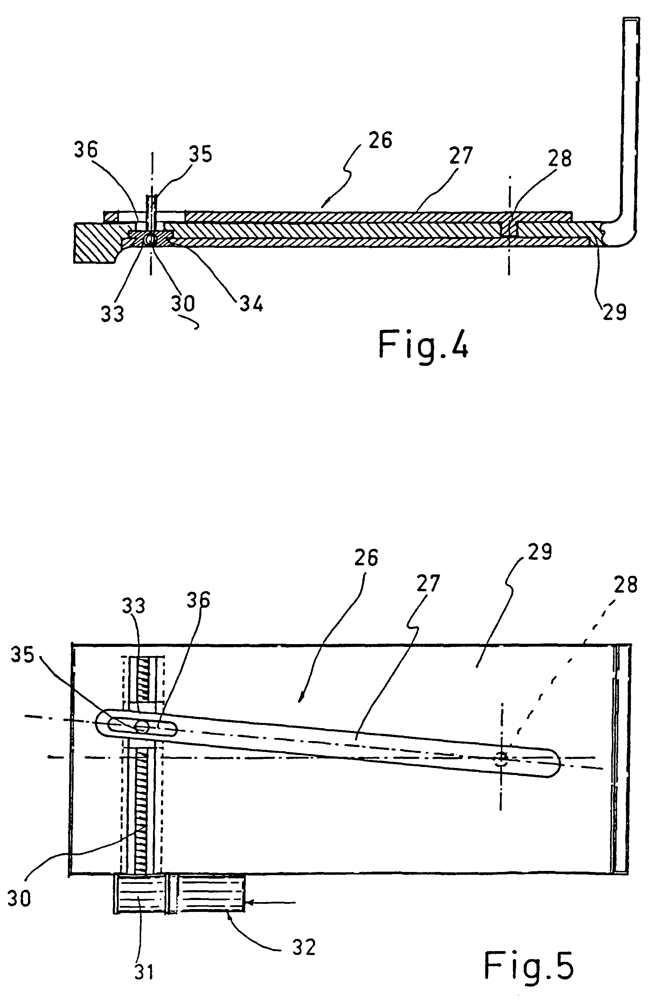

By means of a device 26 shown in detail in Figures 4 and 5, at least one of piston pumps 12, 13 may be displaced angularly to vary respective angle α, β as required by the operator.

As respective devices 26 of piston pumps 12 and 13 are identical, the following description deals solely with device 26 activating piston pump 13 integral with a supporting table 27 hinged at 28 to a base 29. A screw 30, activated by a step motor 32 via a reducer 31, moves a slide 33, which, by means of a dove-tail connection 34, moves together with base 29. The upper surface of slide 33 supports a pin 35 engaging a slot 36 formed in supporting table 27; and, as slide 33 moves, pin 35 pushes against the longitudinal lateral surfaces of slot 36 to rotate supporting table 27, which, as stated, is hinged at 28 to base 29. Angular displacement of supporting table 27, i.e. a variation in angle β, also rotates piston pump 13 integral with table 27, thus enabling the operator to regulate the intake by piston pump 13 of the dialysis fluid flowing along fourth conduit 10.

The Figure 4 and 5 device 26 is shown purely by way of example, it being obvious that any equivalent mechanism may be employed in alternative embodiments of the present invention, providing it ensures step-by-step angular displacement of at least one of piston pumps 12 and 13.

Similarly, though the Figure 1 to 3 solution comprises one electric motor 14 for reciprocatingly operating the pistons of both pumps 12 and 13, each piston pump 12, 13 may obviously be operated by a respective electric motor, in which case, angles α and β are defined by the longitudinal axes of symmetry 20, 21 of piston pumps 12, 13 intersecting the longitudinal axes of symmetry of the respective electric motors (not shown in the accompanying drawings).

The weight loss of the patient is set by the operator as follows.
(a) Firstly, the flow rate of dialysis fluid along first conduit 3 must be made equal to that along fourth conduit 10. If a single electric motor 14 is used for two identical piston pumps 12, 13, this is done by simply determining longitudinal axes of symmetry 20, 21 define equal angles α and β with respect to longitudinal axis of symmetry 19 of electric motor 14, so that the amount of dialysis fluid drawn by piston pump 13 definitely equals the amount compressed by piston pump 12.
(b) By appropriately increasing angle β on the basis of correlation diagrams not shown, and leaving angle α unchanged, intake by piston pump 13 is increased with respect to compression by piston pump 12; piston pump 13 therefore draws in more fluid from dialyzer 4 along fourth conduit 10 than is fed into the dialyzer along first conduit 3; and a vacuum is formed in dialyzer 4 to induce the toxic substances in the blood flowing along second channel 7 to diffuse from channel 7 through permeable membrane 5 into first channel 6.

The more angle β is increased with respect to angle α, the greater the weight loss of the patient will be, by increasing the intake of toxic substances contained in the blood flowing along second channel 7.

Each variation of angle β may therefore be made by activating step motor 32 to rotate reducer 31, which in turn moves slide 33 via screw 30, so that pin 35, integral with slide 33, engages the lateral walls of slot 36 formed in supporting table 27 of second piston pump 13, thus rotating pump 13.

Unit 1 according to the present invention also comprises an electronic central control unit 37 for automatically controlling all the above operations.

The dot-and-dash block 38 in Figure 1 represents an auxiliary weighing device for tapping the dialysis fluid.

Auxiliary weighing device 38 comprises a fifth conduit 39, which is connected at 40 to fourth conduit 10 to feed the dialysis fluid into a vessel 41 by means of a peristaltic pump 42. Again with reference to Figure 1, number 43 indicates electronic weighing means for weighing vessel 41 and its contents, and 44 indicates a solenoid valve fitted along a sixth conduit 45 to a drain 46.

After equalizing flow through both piston pumps 12 and 13, the operator activates auxiliary weighing device 38 to set a given weight loss of the patient by means of peristaltic pump 42. An increase in the speed of peristaltic pump 42 obviously increases the weight loss of the patient; and electronic weighing means 43 provide for retroactively controlling the weight loss set by the operator.

Unit 1 as described provides for maximum freedom in establishing the best conditions in which to determine the weight loss of the patient in each individual case.

It should be stressed that block 38 may be used in conjunction with piston pumps 12, 13 operating solely as flow equalizers, or piston pumps 12, 13 may be used alone, as described previously, in which case, peristaltic pump 42 acts as a valve for closing fifth conduit 39.

## Claims

1. A unit (1) for dialysis treatment, the unit (1) comprising:
a dialyzer (4) in which a membrane (5) defines two channels (6, 7), a first (6) for dialysis fluid and a second (7) for extracorporeal blood;
a first conduit (3) along which said dialysis fluid flows to said first channel (6) of said dialyzer (4);
a second conduit (10) along which said dialysis fluid flows from said first channel (6) of said dialyzer (4); and
at least two piston pumps (12, 13);
the unit (1) being **characterized in that** said at least two piston pumps (12, 13) act on the dialysis fluid in said first conduit (3) and said second conduit (10) respectively; said at least two piston pumps (12, 13) being activated by at least one electric motor (14) via respective universal joints (17, 18);
and being **characterized by** also comprising means (26) for varying at least one angle (α, β) defined by the longitudinal axis of symmetry (19) of said electric motor (14) intersecting the longitudinal axis of symmetry (20) of a first said piston pump (12) or, respectively, the longitudinal axis of symmetry (21) of a second said piston pump (13).

2. A unit (1) as claimed in Claim 1, wherein said means (26) for varying said at least one angle (α, β) comprise:
(a) a screw (30) rotated by a motor (32) via a reducer (31); and
(b) a slide (33) translated by said screw (30) and having a pin (35) engaging a slot (36) formed in a supporting table (27) supporting at least one said piston pump (12, 13); said supporting table (27) being hinged (28) to a base (29).

3. A unit (1) as claimed in Claim 1, wherein said piston pumps (12, 13) are activated by two separate electric motors.

4. A unit (1) as claimed in Claim 1, wherein control is performed entirely by an electronic central control unit (37).

5. A unit (1) as claimed in Claim 1 and further comprising a block (38) comprising at least one peristaltic pump (42), which, by means of a fifth conduit (39), feeds the dialysis fluid into a vessel (41) which is weighed by electronic weighing means (43).

## Patentansprüche

1. Einheit (1) für die Dialysebehandlung, wobei die Einheit (1) umfaßt:
einen Dialysator (4), in dem eine Membran (5) zwei Kanäle (6, 7) definiert, einen ersten Kanal (6) für eine Dialyseflüssigkeit und einen zweiten Kanal (7) für extracorporales Blut;
eine erste Leitung (3), durch die die Dialyseflüssigkeit zum ersten Kanal (6) des Dialysators (4) fließt;
eine zweite Leitung (10), durch die die Dialyseflüssigkeit von dem ersten Kanal (6) des Dialysators (4) fließt; und
wenigstens zwei Kolbenpumpen (12, 13);
**dadurch gekennzeichnet, daß** die wenigstens zwei Kolbenpumpen (12, 13) auf die Dialyseflüssigkeit in der ersten Leitung (3) bzw. der zweiten Leitung (10) wirken, wobei die wenigstens zwei Kolbenpumpen (12, 13) durch wenigstens einen elektrischen Motor (14) mittels jeweiliger Universalgelenke (17, 18) betätigt werden;
und Mittel (26) zum Varieren wenigstens eines Winkels (α, β), der durch die longitudinale Symetrieachse (19) des elektrischen Motors (14), die sich mit der longitudinalen Symetrieachse (20) einer ersten Kolbenpumpe (12) bzw. der longitudinalen Symetrieachse (21) einer zweiten Kolbenpumpe (13) schneidet, definiert ist.

2. Einheit (1) nach Anspruch 1, wobei das Mittel (26) zum Variieren des wenigstens einen Winkels (α, β) umfaßt:
(a) eine Schnecke (30), die durch einen Motor (32) über einen Reduzierer (31) gedreht wird; und
(b) einen Gleitblock (33), der durch die Schnecke (30) translatorisch bewegt wird und einen Stift (35) aufweist, der in einen Schlitz (36) eingreift, welcher in einem Abstütztisch (27) ausgebildet ist, der wenigstens eine der Kolbenpumpen (12, 13) abstützt, wobei der Abstütztisch (27) mit einer Basis (29) gelenkig (28) verbunden ist.

3. Einheit (1) nach Anspruch 1, wobei die Kolbenpumpen (12, 13) durch zwei getrennte elektrische Motoren betrieben werden.

4. Einheit (1) nach Anspruch 1, wobei die Kontrolle komplett von einer elektronischen zentralen Kontrolleinheit (37) ausgeführt wird.

5. Einheit (1) nach Anspruch 1, die weiter einen Block (38) umfaßt, der wenigstens eine Schlauchradpumpe (42) umfaßt, die mittels einer fünften Leitung (39) die Dialyseflüssigkeit in einen Kessel (41) befördert, der durch ein elektronisches Wegemittel (43) gewogen wird.

## Revendications

1. Unité (1) de traitement par dialyse, l'unité (1) comprenant :
un dialyseur (4) dans lequel une membrane (5) définit deux canaux (6, 7), un premier (6) pour le fluide dialyse et un deuxième (7) pour le sang extra-corporel ;
un premier conduit (3) dans lequel ledit fluide de dialyse s'écoule vers ledit premier canal (6) dudit dialyseur (4) ;
un deuxième conduit (10) dans lequel ledit fluide de dialyse s'écoule depuis ledit premier canal (6) dudit dialyseur (4) ; et
au moins deux pompes à piston (12, 13) ;
l'unité (1) étant **caractérisée en ce que** lesdites au moins deux pompes à piston (12, 13) agissent sur le fluide dialyse dans respectivement ledit premier conduit (3) et ledit deuxième conduit (10) ; lesdites au moins deux pompes à piston (12, 13) étant activées par au moins un moteur électrique (14) par l'intermédiaire de liaisons à rotule (17, 18) ;
et étant **caractérisée en ce qu'**elle comprend aussi un moyen (26) destiné à faire varier au moins un angle (α, β) défini par l'axe longitudinal de symétrie (19) dudit moteur électrique (14) coupant l'axe longitudinal de symétrie (20) d'une première pompe à piston (12) ou, respectivement, l'axe longitudinal de symétrie (21) d'une deuxième pompe à piston (13).

2. Unité (1) selon la revendication 1, dans laquelle ledit moyen (26) de variation d'au moins un angle (α, β) comprend :
(a) une vis (30) que fait tourner un moteur (32) par l'intermédiaire d'un réducteur (31) ; et
(b) un coulisseau (33) translaté par ladite vis (30) et comportant une goupille (35) s'engageant dans une fente (36) formée dans une table support (27) portant au moins l'une desdites pompes à piston (12, 13) ; ladite table support (27) étant articulée (28) avec une base (29).

3. Unité (1) selon la revendication 1, dans laquelle lesdites pompes à piston (12, 13) sont activées par des moteurs électriques séparés.

4. Unité (1) selon la revendication 1, dans laquelle la commande est entièrement exécutée par une unité électronique centrale de commande (37).

5. Unité (1) selon la revendication 1, dans laquelle et comprenant en outre un bloc (38) comprenant au moins une pompe péristaltique (42) qui, au moyen d'un cinquième conduit (39), amène le fluide de dialyse dans un réservoir (41) qui est pesé par un moyen électronique de pesée (43).
